# EUROPEAN PATENT SPECIFICATION

(11) **EP 0 280 347 B1**
(45) Date of publication and mention of the grant of the patent: **31.03.1993**
(21) Application number: 88200158.9
(22) Date of filing: 29.01.1988
(51) Int. Cl.: A61B 5/14

(54) **Device for sucking up and releasing a liquid drop by drop**
Vorrichtung zum tropfenweisen Aufsaugen und Abgeben einer Flüssigkeit
Dispositif d'aspiration et de décharge d'un liquide goutte à goutte

(30) Priority: 05.02.1987 NL 8700277
(43) Date of publication of application: 31.08.1988
(73) Proprietor: LIVESTOCK CONTROL HOLDING B.V., NL-3962 CJ WIJK BIJ Duurstede (NL); MICROPLAST B.V., NL-8200 AP Lelystad (NL)
(72) Inventor: Van der Mei, Jacob, NL-3511 NT Utrecht (NL); Dongelmans, Johannes Cornelis, NL-3815 PB Amersfoort (NL)
(74) Representative: Baarslag, Aldert D.

(56) References cited:
- EP-A- 0 002 038
- DE-A- 2 439 218
- FR-A- 1 442 456
- US-A- 4 003 262

## Description

The invention relates to a device for sucking up and releasing a liquid drop by drop.

Such a device is used in particular for sucking up blood, for example obtained through an incision in the skin of a living being, and subsequently obtaining one or more drops of this blood for carrying out a test or tests, for example for determining the composition of the blood.

A known device of this type consists of a pipette with a balloon connected thereto. When the balloon is squeezed by hand and then gradually released, while the rather pointed end of the pipette is dipped in the blood, a quantity of blood can be sucked up into the pipette, after which one or more drops of blood can be expelled from the pipette by squeezing the balloon again.

The disadvantage of this device is that the size of the drops obtained depends on the force with which the balloon is squeezed, so that it is practically impossible to obtain drops of constant size, which is generally a requirement for carrying out the tests. Besides, it is already difficult per se to expel a single drop of blood from the pipette every time with the known device.

Further EP-A-0002038 discloses a capillary vessel comprising a vessel and a member having a funnel-shaped portion which forms the capillar which member is detachably connected to said vessel. When the capillar is dipped in a quantity of blood said blood will be sucked up by the capillary force but soon afterwards will flow by gravity into the vessel without being impeded by the capillary force.

The object of the invention is to produce a device of the known type which does not have these disadvantages.

This object is achieved in that the device according to the invention comprises a first capillary space with at least one inlet aperture, said space being connected to at least a second smaller capillary space having at least one outlet aperture, while the first capillary space is formed in such a way that when the inlet aperture is dipped in the liquid in question a certain minimum quantity of the liquid can be sucked up into said space, and the second capillary space is formed in such a way that when the device is placed with the outlet aperture pointing downwards a quantity of the liquid in question can be retained in said second space through the capillary forces, the later quantity being smaller than the first-mentioned minimum quantity, while the outlet aperture is designed in such a way that the quantity of liquid which is the difference between the first and second quantity can flow out of the second capillary space essentially in drop form.

With a device in this way capillary forces are thus used exclusively both for sucking up a quantity of blood and for obtaining one or more drops of blood for taking samples, so that during the formation of the drops no mechanical forces and/or shape changes occur, with the result that the size of the drops is not affected by other factors and is therefore highly constant. Moreover, air bubbles do not occur, which helps to keep the drop size constant.

The first capillary space is preferably elongated in cross section, with a longer longitudinal elongated and a shorter transverse measurement at right angles thereto, while at least the part of the second capillary space adjoining the outflow aperture is of a cross-sectional shape which has essentially equal longitudinal and transverse measurements virtually of the same order of size as the transverse measurement of the first capillary space.

In an embodiment which is simple to produce the first capillary space is rectangular in transverse section and the above-mentioned part of the second capillary space is in the form of a square in cross section, the capillary space which is elongated in cross section preferably merging gradually into the above-mentioned part of the second capillary space.

A collection container can be advantageously connected to the outlet aperture, said container being detachably connected to the device.

When a quantity of blood has been sucked into the device, said container can be filled with the blood flowing in drops from the outflow aperture and can then be removed from the device, so that a quantity of blood corresponding exactly to the capacity of the container is obtained.

The invention will now be explained in greater detail with reference to the drawing, in which the embodiments of the device according to the invention are illustrated.

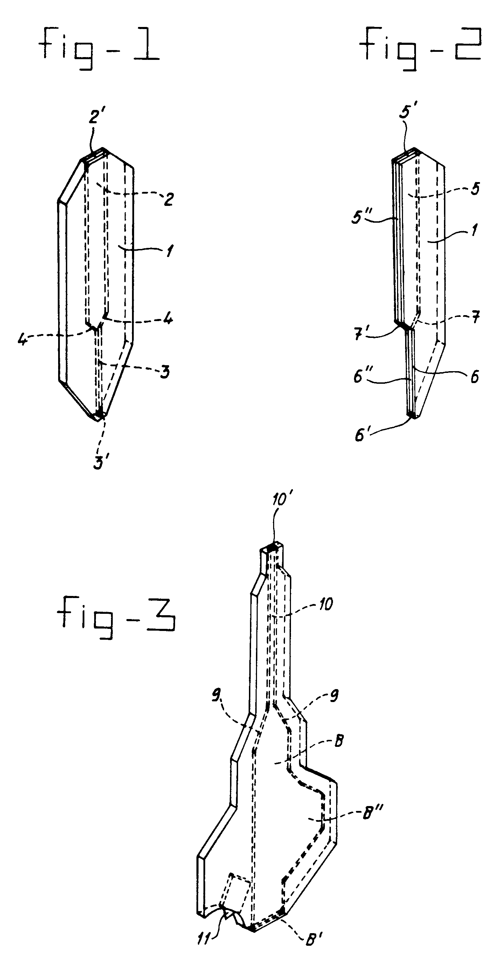

As shown in figure 1, the device comprises a flat body 1, for example made of plastic, metal, glass or similar material.

A first capillary space 2 is formed inside the body and has an inlet aperture 2ʹ. Said capillary space 2 has a cross section which is in the form of a rectangle with a longitudinal measurement of, for example, 5 mm and a transverse measurement of, for example, 0.7 mm, the length of the space being approximately 20 mm.

The body 1 also contains a second capillary space 3 with an outlet aperture 3ʹ. The space 3 is, for example, 25 mm in length and is square in cross section with, for example, measurements of 0.7 by 0.7 mm. Said space 3 gradually merges with the space 2 via the slanting walls 4.

When the device is in use the inlet aperture 2ʹ is dipped in a quantity of blood which is obtained, for example, through an incision in the skin, this inlet aperature 2ʹ being pointed downwards. A quantity of the blood is then sucked up into the capillary space 2. So long as the inlet aperture is in contact with the blood, blood will flow in until a state of equilibrium has been reached between gravity and the capillary forces. The body 1 is then turned over to the position shown in Figure 1, so that the quantity of blood in the space 2 flows downwards into the capillary space 3 and in drop form out of the outflow aperture 3ʹ, until a state of equilibrium has come about again between gravity and the capillary forces. The quantity of blood sucked up into the space 2 is greater here than the quantity of blood with which the latter-mentioned state of equilibrium is reached, so that at least one drop of blood comes out of the outflow aperature 3ʹ.

A collection container, not shown, connected to the outlet aperture 3ʹ, can be connected detachably, preferably in tear-off form, to the body 1. When it is full of blood dripping out of the outlet aperature 3ʹ, this container can then be torn off the body 1, so that an accurate quantity of blood corresponding to the capacity of the container is obtained, which can be smaller than one drop.

The embodiment shown in figure 2 comprises substantially one half of the device shown in figure 1 so that of the first and second capillary spaces 5 and 6 respectively the one sides 5ʺ and 6ʺ are open and the inlet and outlet apertures 5ʹ and 6ʹ respectively are bounded by only three sides of a rectangle and a square respectively, whereas one 7ʹ of the slanting walls 7 is also cut open.

In the embodiment shown in figure 3 the first capillary space 8 has a laterally protruding portion 8ʺ spaced apart from the inlet orifice 8ʹ and from the slanting walls 9 via which said first capillary space 8 merges with the second capillary space 10 having outlet orifice 10ʹ. A sharp metal cutting member 11 for making an incision in the skin of a living being is mounted in a recess formed in the portion adjacent to the first capillary space 8.

## Claims

1. Device for sucking up and releasing a liquid drop by drop, the device comprising a first capillary space (2) with at least one inlet aperture, (2') characterized in that said space being connected to at least a second smaller capillary space (3) having at least one outlet aperture (3'), while the first capillary space being formed in such a way that when the inlet aperture (2') is dipped in the liquid in question a certain minimum quantity of the liquid can be sucked up into said space, and the second capillary space (3) is formed in such a way that when the device is placed with the outlet aperture (3') pointing downwards a quantity of the liquid in question can be retained in said second space (3) through capillary forces, the latter quantity being smaller than the first-mentioned minimum quantity, while the outlet aperture (3') is designed in such a way that the quantity of liquid which is the difference between the first and second quantity can flow out of the second capillary space (3) essentially in drop form.

2. Device according to claim 1, characterized in that the first capillary space is elongated in cross section, with a longer longitudinal measurement and a shorter transverse measurement at right angles thereto, while at least the part of the second capillary space adjoining the outflow aperture is of a cross sectional shape which has essentially equal longitudinal and transverse measurements virtually of the same orer of size as the transverse measurement of the first capillary space.

3. Device according to claim 2, characterized in that at least one of said capillary spaces is open at one of its short sides corresponding with said shorter transverse measurement.

4. Device according to claim 2, characterized in that the first capillary space has a cross section which is in the form of a rectangle, and the above-mentioned part of the second capillary space has a cross section which is in the form of a square.

5. Device according to claims 2-4, characterized in that the capillary space with elongated cross section gradually merges into the above-mentioned part of the second capillary space.

6. Device according to claims 1-5, characterized in that a collection container is connected to the outlet aperture and is detachably connected to the device.

## Patentansprüche

1. Vorrichtung zum tropfenweisen Aufsaugen und Abgeben einer Flüssigkeit, welche einen ersten Kapillarraum (2) mit mindestens einer Eintrittsöffnung (2') aufweist, dadurch gekennzeichnet, daß der Raum mit mindestens einem zweiten kleineren Kapillarraum (3) verbunden ist, der mindestens eine Austrittsöffnung (3') aufweist, wobei der erste Kapillarraum derart ausgebildet ist, daß dann, wenn die Eintrittsöffnung (2') in die fragliche Flüssigkeit eingetaucht wird, eine bestimmte Mindestmenge der Flüssigkeit in den Raum hochgesaugt werden kann, und der zweite Kapillarraum (3) derart ausgebildet ist, daß dann, wenn die Vorrichtung mit der Austrittsöffnung (3') nach unten gerichtet angeordnet wird, eine Menge der fraglichen Flüssigkeit durch Kapillarkräfte in dem zweiten Raum (3) gehalten werden kann, wobei die letztere Flüssigkeitsmenge kleiner ist als die erste Mindestmenge, und wobei die Austrittsöffnung (3') derart ausgebildet ist, daß die Flüssigkeitsmenge, die den Unterschied zwischen der ersten und der zweiten Menge bildet, im wesentlichen in Tropfenform aus dem zweiten Kapillarraum (3) herausfließen kann.

2. Vorrichtung gemäß Anspruch 1, dadurch gekennzeichnet, daß der erste Kapillarraum einen länglichen Querschnitt aufweist, wobei eine längere Längsabmessung und eine kürzere Querabmessung im rechten Winkel zueinander stehen, wobei mindestens der der Ausflußöffnung benachbarte Teil des zweiten Kapillarraums eine Querschnittsform aufweist, die im wesentlichen gleiche Längs- und Querabmessungen aufweist, die grundsätzlich die gleiche Größenordnung haben, wie die Querabmessung des ersten Kapillarraums.

3. Vorrichtung gemäß Anspruch 2, dadurch gekennzeichnet, daß mindestens einer der Kapillarräume an einer seiner kurzen Seiten offen ist, welche der kürzeren Querabmessung entspricht.

4. Vorrichtung gemäß Anspruch 2, dadurch gekennzeichnet, daß der erste Kapillarraum einen Querschnitt aufweist, der die Form eines Rechtecks hat, und der vorstehend genannte Teil des zweiten Kapillarraums einen Querschnitt in Form eines Quadrats hat.

5. Vorrichtung gemäß Ansprüchen 2 bis 4, dadurch gekennzeichnet, daß der Kapillarraum mit länglichem Querschnitt allmählich in den vorstehend erwähnten Teil des zweiten Kapillarraums übergeht.

6. Vorrichtung gemäß Ansprüchen 1 bis 5, dadurch gekennzeichnet, daß ein Sammelbehälter mit der Austrittsöffnung verbunden und lösbar mit der Vorrichtung verbunden ist.

## Revendications

1. Dispositif pour aspirer et décharger un liquide goutte à goutte, ce dispositif comportant un premier espace capillaire (2) avec au moins un orifice d'entrée (2'), **caractérisé** en ce que ledit espace est relié à au moins un second espace capillaire (3) plus petit qui comporte au moins un orifice de sortie (3'), sachant que le premier espace capillaire est formé de telle sorte que, quand on plonge l'orifice d'entrée (2') dans le liquide en question, on peut aspirer une certaine quantité minimale de liquide dans ledit espace, et le second espace capillaire (3) est formé de telle sorte que, quand le dispositif est placé avec l'orifice de sortie (3') orienté vers le bas, une quantité de liquide en question puisse être retenue dans ledit second espace (3) par les forces de capillarité, cette dernière quantité étant plus petite que la quantité minimale d'abord mentionnée, alors que l'orifice de sortie (3') est conçu de telle sorte que la quantité de liquide égale à la différence entre la première et la seconde quantités puisse s'écouler du second espace capillaire (3) essentiellement sous forme de goutte.

2. Dispositif selon la revendication 1, **caractérisé** en ce que le premier espace capillaire a une section transversale allongée avec un côté longitudinal plus grand et un côté transversal plus petit et perpendiculaire, alors que, au moins la partie du deuxième espace capillaire qui jouxte l'orifice de sortie a une forme en section transversale essentiellement égale dans le sens transversal et le sens longitudinal et virtuellement du même ordre de grandeur que la mesure transversale du premier espace capillaire.

3. Dispositif selon la revendication 2, **caractérisé** en ce que, au moins l'un desdits espaces capillaires est ouvert au niveau de l'un de ses côtés courts correspondant à ladite mesure transversale la plus courte.

4. Dispositif selon la revendication 2, **caractérisé** en ce que le premier espace capillaire présente une section transversale qui a la forme d'un rectangle, et la partie ci-dessus mentionnée du second espace capillaire présente une section transversale qui a la forme d'un carré.

5. Dispositif selon les revendications 2 à 4, **caractérisé** en ce que l'espace capillaire avec la section transversale allongée se fond progressivement avec la partie ci-dessus mentionnée du second espace capillaire.

6. Dispositif selon les revendications 1 à 5, **caractérisé** en ce qu'un réservoir d'accummulation est relié à l'orifice de sortie et est relié de façon détachable au dispositif.
